(19) Europäisches Patentamt
European Patent Office
Office européen des brevets

(11) Publication number : **0 330 695 B1**

(12) # EUROPEAN PATENT SPECIFICATION

(45) Date of publication of patent specification :
**11.11.92 Bulletin 92/46**

(51) Int. Cl.$^5$ : **C12P 13/02,** C12P 17/10,
// C12P41/00

(21) Application number : **88907648.5**

(22) Date of filing : **17.08.88**

(86) International application number :
**PCT/DK88/00134**

(87) International publication number :
**WO 89/01525 23.02.89 Gazette 89/05**

(54) **PROCESS FOR PREPARATION OF ORGANIC CHEMICALS.**

Consolidated with 88201761.9/0307023 (European application No./publication No.) by decision dated 23.05.90.

(30) Priority : **17.08.87 DK 4268/87**
**10.11.87 DK 5861/87**
**03.08.88 NL 8802014**

(43) Date of publication of application :
**06.09.89 Bulletin 89/36**

(45) Publication of the grant of the patent :
**11.11.92 Bulletin 92/46**

(84) Designated Contracting States :
**AT BE CH DE ES FR GB GR IT LI LU NL SE**

(56) References cited :
**EP-A- 0 193 113**
**EP-A- 0 199 407**
**FR-A- 2 586 702**
**Chemical Abstracts, Volume 74, No. 3, 18 January 1971, (Columbus, Ohio, US), see page 37, abstract 9894e, & JP, a,7020958 (TANABE SEIYAKU CO. LTD) 16 July 1970**

(73) Proprietor : **NOVO INDUSTRI A/S**
**Novo Allé**
**DK-2880 Bagsvaerd (DK)**
Proprietor : **STAMICARBON B.V.**
**Mijnweg 1**
**NL-6167 AC Geleen (NL)**

(72) Inventor : **GODTFREDSEN, Sven, Erik**
**15 B Smedegade**
**DK-3500 Vaerloese (DK)**
Inventor : **CLAUSEN, Kim**
**85 Rodlersvej**
**DK-2990 Niv (DK)**
Inventor : **INGVORSEN, Kjeld**
**35 Klostergardsvej**
**DK-3500 Vaerloese (DK)**
Inventor : **HERMES, Hubertus, Fransiscus, Maria**
**59 Overstraat**
**NL-6151 CM Sittard (NL)**
Inventor : **VAN BALKEN, Johannes, Arnoldus, Maria**
**1 Schrijnwerkerstraat**
**NL-6114 XH Susteren (NL)**
Inventor : **MEIJER, Emmo, Marinus**
**12 Merelstraat**
**NL-6167 EZ Beek (NL)**

(74) Representative : **Hoogstraten, Willem Cornelis Roeland et al**
**OCTROOIBUREAU DSM Postbus 9**
**NL-6160 MA Geleen (NL)**

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Jouve, 18, rue Saint-Denis, 75001 PARIS

## Description

The present invention relates to a process for complete or partial racemization of compounds of the general Formula I

$$R\text{-}CH(NH_2)\text{-}CONH_2 \quad (I)$$

wherein R represents indolyl, benzyloxy, lower alkyl optionally substituted by hydroxy, mercapto, amino, halogen, phenyl, phenoxy, benzyl or lower alkylthio, or R represents phenyl optionally substituted by one or more of the following substituents: hydroxy, amino, halogen, carboxy or lower alkoxy.

## BACKGROUND OF THE INVENTION

Amino acid amides are readily obtainable compounds by chemical as well as by enzymatical processes, and are as such important as precursors in the production of amino acids. Optically active amino acids can be obtained by e.g. enantioselective enzymatic hydrolysis of amino acid amides. Simultaneous enzymatical racemization of the amino acid amides, which is thusfar unknown, will be of great advantage because then complete conversion of the amino acid amide into the desired optically active amino acid is possible.

As used in this specification, a stereoselective (enantioselective) enzyme is one that reacts exclusively or preferentially with one of two enantiomers, thereby producing an optically pure or optically active product, respectively. A stereospecific (enantiospecific) enzyme is one that reacts exclusively with one of the enantiomers to produce an optically pure product.

Optically active α-H, α-amino acids constitute a class of organic compounds of great industrial importance. Two major groups of these compounds can be identified: the natural amino acids found in nature e.g. as constituents of proteins, peptides and a wide variety of other important compounds, and the so-called unnatural amino acids which share certain characteristic structural features with the naturally occurring amino acids but which are not natural constituents of living matter. Both groups of amino acids find important industrial applications. The naturally occurring amino acids are thus applied abundantly as food and feed additives and in the pharmaceutical industry e.g. as components of infusion liquids. In recent years, also unnatural amino acids have found extensive uses for example as intermediates for various pharmacological compounds.

Due to their molecular structure amino acids of Formula I can occur in two distinct forms differing in respect to the so-called chirality of the amino acid molecules. These two forms of a given amino acid are usually denoted as the D- or the L-form of the amino acid. Most amino acids found in nature are of the L-configuration and it has turned out to be essential that amino acids used for food and feed additives are of this configuration since the corresponding D-isomers cannot, usually, be metabolised by living cells and will even interfere with normal cell metabolism and function. In consequence of this, these unnatural amino acids can often be used as building blocks for pharmaceutical and agrochemical products. For example, D-phenylglycine and D-p-hydroxyphenylglycine are used for broad spectrum antibiotics such as Ampicillin and Amoxycillin, respectively.

Because of the growing demand for selective products in the pharmaceutical, food and agrochemical industries it is highly desirable to have available methods for producing optically pure, i.e. enantiomerically pure, amino acids of the D- as well as of the L-configuration while, on the contrary, mixtures of the two enantiomeric forms of a given amino acid, which are denoted racemates when the two forms are present in equal quantities, are of a more limited industrial interest. The reason for this is that only one of the two optical isomers exhibits a clear activity in the desired applications. The other optical isomer, however, may exhibit no activity, hardly any activity, or even undesired side activity.

Known processes for preparing optically active compounds are e.g. fermentation, asymmetrical synthesis and chemical or enzymatic resolution processes. They have been described in reviews, e.g. by G. Schmidt-Kastner and P. Egerer in Biotechnology, H.-J. Rehm and G. Reed (Eds.), Verlag Chemie, Florida-Basel, Vol. 6a, pp. 387-421 (1984) and by E.M. Meijer et al. in Symposium Proceedings: "Biocatalysts in Organic Syntheses", Noordwijkerhout, April 14-17, 1985, Elsevier Science Publishers.

For the production of L-amino acids, in particular microbial fermentation is a suitable preparation method, besides a number of enzymatic resolution methods and enzymatic asymmetrical syntheses. Thus, today an increasing number of natural amino acids are produced by fermentation techniques which, however, cannot be applied for production of unnatural amino acids, whether these are D-forms of the naturally occurring L-amino acids (with exception of a few naturally occurring D-amino acids, e.g. D-alanine) or amino acids which by virtue of their chemical composition (depending on the R-substituent) do not occur in nature.

For the preparation of these unnatural amino acids the above-mentioned processes such as asymmetrical synthesis and chemical or enzymatical resolution can be used.

In recent years there has been an increasing interest in applying enzymes in organic synthesis in general. The developments in this area are due to the recognition that enzymes, although occurring and applied in nature

as biocatalysts for synthesis of naturally occurring compounds, do catalyze a variety of reactions which usually do not take place in nature but which can nevertheless be utilised to advantage by the organic chemist. These developments regarding the use of enzymes for synthesis are of particular interest in connection with amino acid production since enzymes, being enantioselective, possess the ability to catalyze formation of optically pure compounds. In such processes the enantioselective properties of enzymes are utilized to generate an optically pure compound by converting only one of the enantiomers present in a racemic mixture typically made available by chemical synthesis.

Besides of the high stereoselectivity, enzymatic processes are preferred in view of the mild reaction conditions and the usually broad substrate specificity. The last-mentioned aspect refers to the fact that one and the same enzyme preparation can be used for the preparation of several different amino acids.

An excellent example of an enzymatic resolution method is described in US-A-3,971,700, US-A-4,080,259 and US-A-4,172,846. In these patents a racemic D,L-amino acid amide is brought into contact with a preparation containing L-enantioselective amidase, as indicated in Scheme I. Only the L-amino acid amide is hydrolyzed, and a mixture of L-amino acid and D-amino acid amide is formed. The L-amino acid can be recovered therefrom by several methods, e.g. by crystallization, by extraction or by ion exchange chromatography. Also, the D-amino acid amide can be precipitated as a Schiff base by reaction with e.g. benzaldehyde. After recovery of the Schiff base, the D-amino acid amide can be recovered and from this the D-amino acid can subsequently be recovered by chemical (acid) hydrolysis as well as by enzymatic hydrolysis (see e.g. EP-A-179,523).

The method disclosed in the above patents serves, therefore, as a means for preparation of optically pure D- as well as L-amino acids. However, it remains a disadvantage that at most 50% of the racemic starting material can be utilized for the recovery of the corresponding D- or L-amino acid.

Scheme I.

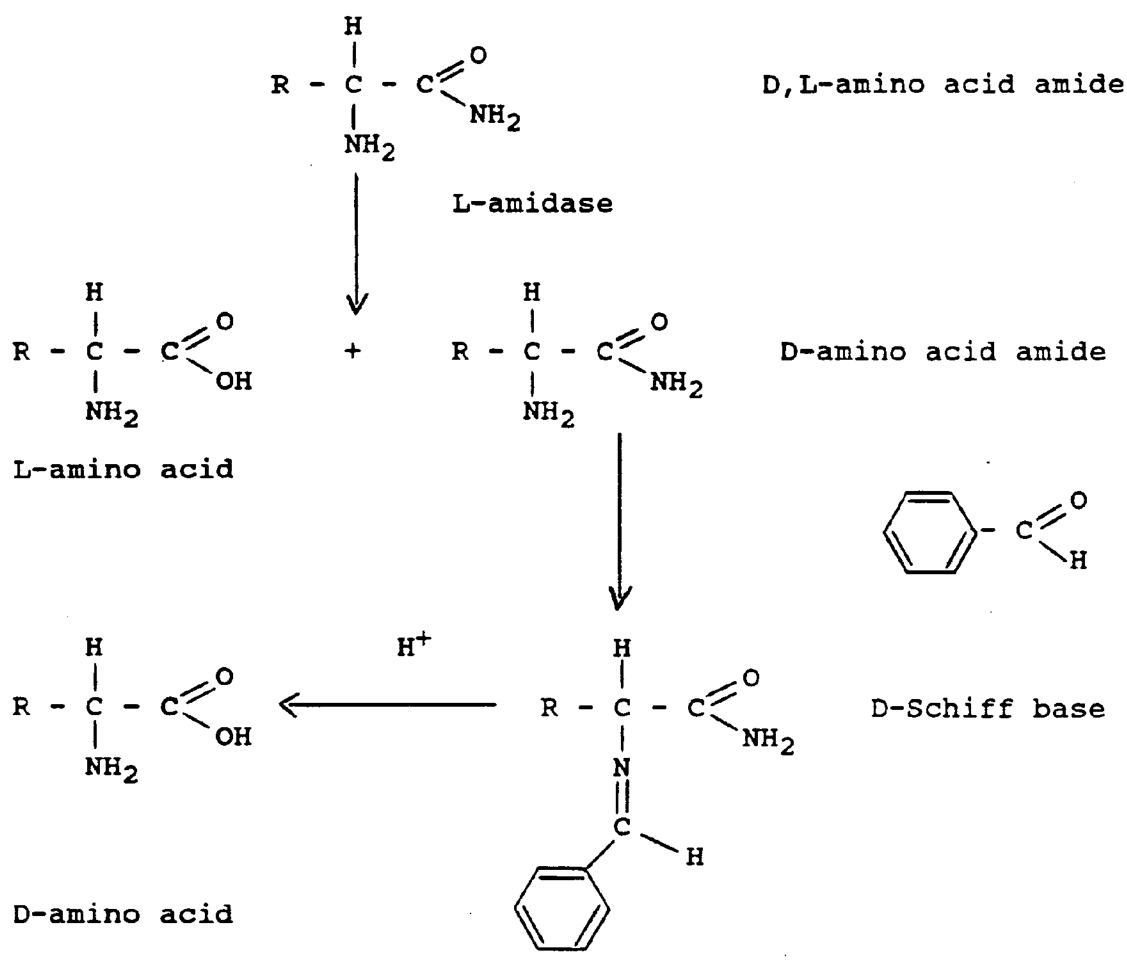


As seen, amides are important precursors for the production of optically active amino acids.

The general principle of utilising enantioselective properties of enzymes to separate a racemic mixture of a precursor for an amino acid into a mixture of the optically pure amino acid and the optically pure precursor has a wide applicability but suffers from at least one important disadvantage: usually only one form of the optically pure amino acid is required in large quantities, there being no need to produce its enantiomeric counterpart. Accordingly, there is usually no need for either the optically pure precursor or the optically active amino acid remaining after production of the amino acid actually needed has been carried out. In industrial practice it is required, therefore, to racemize and recycle unused material when applying the principle of enantioselective enzymatic synthesis for production of organic chemicals. In many instances racemization of precursor molecules remaining after conversion of a racemic mixture of the precursor is thus an integral part of enzymatic synthesis of optically pure amino acids. Accordingly, quite some attention has been devoted to this problem there being available a number of chemical methods for racemization of amino acid precursors.

For example, D-amino acid amides have been found to be readily racemizable by conversion into their D-N-benzylidene derivatives (see US-A-4,094,904 and US-A-4,172,846).

Chemical methods for racemization of amino acid amides are, however, usually not compatible with enzymatic reactions, one consequence being that the racemization and resolution steps have to be carried out separately. Moreover, chemical methods for racemization of amino acid amide precursors suffer from the disadvantage that they impose an extra cost onto the overall process due to the cost of chemicals, equipment, losses during the racemization step by e.g. by-product formation and a variety of other circumstances.

## STATEMENT OF THE INVENTION

Surprisingly, we have discovered the existence of enzymes with amino acid amide racemase activity, which was previously unknown. Accordingly, the invention provides a process for racemization of amino acid amides of the general Formula I characterized by exposing said compounds to an enzyme of microbial origin with amino acid amide racemase activity towards the amide in question.

Numerous advantages can be gained by applying the process of the invention for racemization of amino acid amides instead of using chemical or other technologies for this conversion. First of all, the mild conditions usually prevailing during enzymatic reactions result in low losses of material through by-product formation. Also, the process of the invention can be applied without any use of costly chemicals which after their use may even give rise to environmental problems e.g. in regard to their disposal. Further, the conditions used in the process of the invention make it possible to combine enzymatic racemization of amino acid amide with enantioselective enzymatic hydrolysis of the D- or L-amide to prepare optically active amino acid in one single step without the need to separate and recycle the unreacted amide.

The compounds which can be fully or partly racemized according to the method of the invention can be represented by the general Formula I

$$R\text{-}CH(NH_2)\text{-}CONH_2 \qquad (I)$$

wherein R represents indolyl, benzyloxy, lower alkyl optionally substituted with hydroxy, mercapto, amino, halogen, phenyl, phenoxy, benzyl or lower alkylthio, or R represents phenyl optionally substituted by one or more of the following substituents: hydroxy, amino, halogen, carboxy or lower alkoxy characterized by exposing said compounds to an enzyme with amino acid amide racemase activity towards the amide in question. Of course, the process of the invention can also be applied to amides of heterocyclic amino acids, where the group R in Formula I is also linked to the $\alpha$-amino group. One example is the natural amino acid proline.

Examples of the group R are as follows: methyl, isopropyl, secondary butyl, phenyl, p-hydroxyphenyl, benzyl, 2-phenylethyl, 1-hydroxyethyl, mercaptomethyl, methylthiomethyl and phenoxymethyl. Furthermore, the phenyl, indolyl and benzyl group may be substituted by one of the following groups: hydroxy, amino, halogen, carboxy, and lower alkoxy. The term "lower alkyl" designates alkyl containing less than 8, preferably less than 5, carbon atoms. Similarly, lower alkoxy contains less than 8, preferably less than 5, carbon atoms.

In a particularly advantageous embodiment, the process of the invention is used to prepare an optically active amino acid from a corresponding amide by simultaneously contacting the amide with amino acid amide racemase and stereoselective D- or L-amidase with activity towards the D- or L-amino acid amide in question. (This amidase enzyme is also sometimes called aminopeptidase). The reactions are shown in Scheme II.

If L-amidase is used, D-amidase should be absent, and vice versa. Further, amino acid racemase with activity toward the amino acid in question should be absent.

The amino acid amide can be supplied in pure D- or L-form or as a mixture of the two, e.g. a racemic mixture. With this process, it is even possible to convert D-amino acid amide into L-amino acid, or L-amide into D-acid. It may be particularly convenient to use a racemic mixture of amides, prepared chemically.

Scheme II

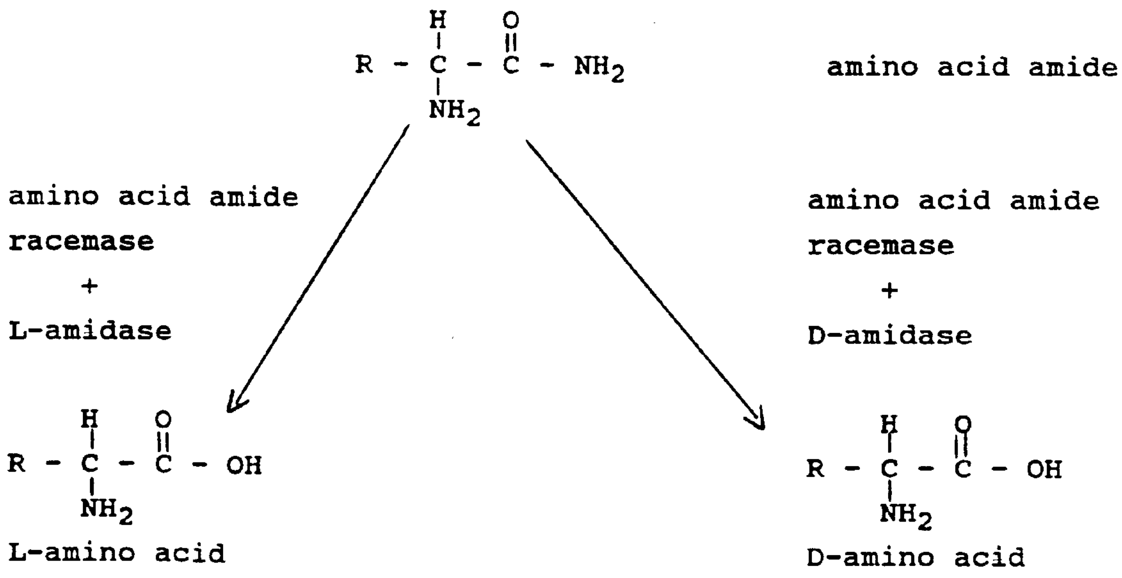


The advantage of this process is that from mixtures of enantiomers, including racemic mixtures, of an amino acid amide, as well as from optically pure D- or L-amino acid amide, an optically pure D- or L-amino acid can be prepared stereo-selectively, the amino acid amide being fully converted to the desired amino acid.

The enzymatic process according to the invention may be carried out, for example, in a batch-wise fashion by stirring a mixture of the amino acid amide to be racemized and the enzyme in an aqueous medium under control of the pH value and the temperature of the reaction mixture. The pH value of the reaction mixture can vary between 6 and 12, in particular between 7.5 and 9.5. The reaction temperature may be between the freezing point of the reaction medium and about 65°C, preferably between 20 and 45°C, and most preferably about 37°C. Outside these ranges the activity and/or the stability of the biocatalyst is generally insufficient for a satisfactory yield. If required, organic solvents may be added to the reaction mixture to increase the solubility of the reactants such solvents being, for example, alcohols such as ethanol, methanol, isopropanol or t-butanol, or other organic solvents like dioxane, N,N-dimethylformamide, dimethylsulfoxide or hexamethylphosphorous triamide. The reaction may also be carried out in a two-phase system using a suspension of reactants or two immiscible solvents like, for example, water and a hydrocarbon like hexane or cyclohexane, or in a fully organic phase of a hydrophobic, water-saturated, organic solvent.

The racemases applied in the process according to the invention may be purified enzymes, a crude enzyme solution, microbial cells exhibiting the required activity, a homogenate of cells or permeabilized cells. If required, the enzyme may be applied in an immobilized state or in a chemically modified form to ensure a good stability and reactivity of the enzyme under the conditions utilized. The process of the invention may further be carried out concomitantly with enantioselective, enzymatic hydrolysis of amino acid amides into amino acids. The above-mentioned enzymes or enzyme activities can also be denoted as biocatalyst(s). This is obviously understood to include a mixture of biocatalysts each containing one of the enzyme activities to be combined, or preferably one biocatalyst in which both enzyme activities are present. The reaction mixture can also be passed through alternating reactors containing the amino acid amide amidase and the racemase enzyme activity, respectively.

Enzymes applied in the process of the invention are enzymes catalyzing conversion of one enantiomer of an amino acid amide into its antipode (or, in case the amide contains additional asymmetric centers, into the epimeric compound).

It is particularly convenient to test enzymes to be applied according to the method of the invention by employing chiral high pressure liquid chromatography (HPLC) as e.g. described in Anal. Biochemistry, 121, 370 (1982) by Weinstein et. al.

The enzymes applied according to the method of the invention may be isolated from plants and animals. Preferably, however, enzymes of microbial origin are used, such microorganisms being bacteria, fungi, or other microorganisms. For example, the following microbial species are potential sources of amino acid amide racemases: species of Pseudomonas, Gluconobacter, Agrobacterium, Acetobacter, Achromobacter, Arthrobacter, Acinetobacter, Alcaligenes, Citrobacter, Enterobacter (such as E. cloaca), Erwinia, Escherichia, Klebsiella,

Proteus, Serratia, Yersinia, Shigella, Peptoccaceae, Pseudomonadadeae, Cytophaga, Bacteroidaseae, Butyrivitrio, Selenomonas, Zymomonas, Chromobacterium, Aeromonas (such as A. proteolytica), Vibrio, Flavobacterium, Micrococcus, Staphylococcus, Streptococcus, Pediococcus, Bacillus (such as B. subtilis and B. stearothermophilus), Clostridium, Lactobacillus, Leuconostoc, Brevibacterium, Thermus, Cellulomonas, Corynebacterium, Microbacterium, Hyphomicrobium, Propionibacterium, Mycobacterium (such as M. phlei) Streptomyces, Bacteridium, Actinomycetales, Chaetomella, Septoria, Doplodia, Phoma, Conothyrium, Myrothecium, Pestalotia, Melaconium, Epicoccum, Penicillium, Rhizopus, Mucor, Candida, Aspergillus ( such as A. oryzae and A. parasiticus), Sepedonium, Fusidium, Oidiodendron, Cephalosporium, Scopulariopsis, Paecilomyces, Verticillium, Tricothecium, Pullularia, Monotospora, Cladosporium, Helmintosporium, Chrysosporium, Rhodotorula, Kloeckera, Saccharomyces, Geotricium, and Fusarium. In particular, the racemase enzyme may be obtained from strains of Pseudomonas (especially Ps. putida) and Rhodococcus, such as the following two strains deposited by the applicants for patenting purposes under the terms of the Budapest Treaty with the National Collections of Industrial & Marine Bacteria Ltd.:

| | | |
|---|---|---|
| Deposit No. | NCIB 12569 | NCIB 40042 |
| Deposit date | 19 Oct. 1987 | 3 Aug. 1988 |
| Depositor | NOVO | DSM |
| Depositor's reference | OD 2111 | 79M |
| Taxonomic designation | Rhodococcus sp. | Ps. putida |

The amino acid amide racemase used in the invention is preferably derived from a strain of one of the above-mentioned microoorganisms, and it can be obtained e.g. by cultivation of the strain in a suitable medium or by cultivation of a transformed host organism containing a gene encoding for and expressing this activity, the gene having been obtained from one of the above organisms.

The suitability of a given strain as a source of amino acid amide racemase or the presence of such an enzyme in crude enzyme preparations is conveniently tested by incubating cells of the microorganism in question or the crude enzyme with the optically pure forms of the amino acid amide of interest. During the incubation aliquots of the reaction mixture are taken and analyzed for the content of amino acid amides and amino acids by employing e.g. chiral HPLC. The occurrence of an amino acid amide racemase will, in those cases where the incubation is performed using the L-amino acid amide, give rise to the presence of the corresponding D-amino acid amide and/or the D-amino acid (when the racemase activity is used concomitantly with a D-amidase activity). Conversely, the L-amino acid amide and/or the L-amino acid (when using concomitantly an L-amidase) will be generated if the D-amino acid amide is incubated with cells or enzymes exhibiting racemase activity.

When carrying out the above-mentioned tests for the presence of an amino acid amide racemase in microorganisms or crude enzymes, amino acid racemases and D- and/or L-amidases will often be encountered and make the interpretation of the experimental results difficult. However, by testing directly for the presence of these amino acid racemases and their activities by incubating optically pure amino acids with the crude enzymes or cells in question some of the difficulties are readily overcome. Alternatively, amino acid racemase inhibitors as e.g. β-chloro-L-alanine may be added to the incubation mixture. Another problem with respect to the interpretation of the experimental results is the presence of D- and/or L-amidase activity simultaneously with the amino acid amide racemase activity. Amino acid amide amidase inhibitors such as α-amino methyl ketones as described by Jahreis et. al. in Biomed. Biochem. Acta. 46, 683 (1987) may be employed to prevent conversion of the amino acid amides to the corresponding amino acids.

Finally, in those instances where analysis of cells or crude enzymes indicates the presence of amino acid amide racemase activity the crude enzymes or the enzymes from the cells tested may be subjected to purification using the methods for protein and enzyme purification well described in the art. Fractions obtained during such purification procedures can be analyzed for amino acid amide racemase activity by employing a chiral HPLC-system.

Microorganisms to be tested for amino acid amide racemase activity may be furnished in a number of independent ways:

1. The microorganisms may be obtained from culture collections. Before test of such organisms it is preferred to expose or grow the microorganism in the presence of e.g. the D-amino acid amide in question. Next to the possibility of induction of D-amidase activity also amino acid amide racemase activity may be induced.

2. The microorganisms may be selected among strains known to produce amino acids of the unnatural configuration, peptides containing amino acids of the unnatural configuration, or compounds derived from amino acids of unnatural configurations. Such microorganisms are listed e.g. in Handbook of Microbiology published by CRC Press Ltd. or in related works.

3. Microorganisms that may produce amino acid amide racemases may also be obtained by exposing an available microorganism to a mutagenic agent such as e.g. NTG, ultra violet radiation, or gamma radiation. Such treatments are known in the art to alter the specificity of enzymes and to induce expression of enzymes from cryptic genes.

4. Microorganisms producing amino acid amide racemases may also be obtained directly by screening employing e.g. enrichment culture techniques. A preferred enrichment method involved addition of environmental soil samples to a suitable enrichment medium supplemented e.g. with the pure D-amino acid amide in question as the sole carbon or nitrogen source. Using this procedure microorganisms producing amino acid amide racemases will be enriched and they can subsequently be isolated by conventional microbiological techniques. A selection pressure can be established during the screening by employing defined and semi-synthetic media. The microorganisms isolated are assayed by employing the methods described above. It is preferred to perform the enrichment under aerobic or anaerobic conditions as well as at different temperatures and pH-values in order to obtain a collection of amino acid amide racemases suitable for use under different conditions.

An illustration of how a strain with D-amidase activity can be furnished is the isolation of the strain NCIB 40041: Erlenmeyer flasks containing 50 ml of an enrichment medium devoid of nitrogen sources but supplemented with D-amide are inoculated with soil samples. After seven days flasks showing good growth are selected and subcultivated in the same medium. After another seven days flasks showing good growth are selected and their content is analyzed for the presence of D-amide. Those cultures devoid of D-amide are spread on agar plates from which colonies are selected and analyzed for amidase activity.

The strain NCIB 40041 was isolated by this procedure. Other D-amidases can be isolated in a similar fashion and their specificity may be influenced by selecting the applied D-amino acid amide in accordance with the need.

In particular, the combination of a biocatalyst with D-amidase activity and a biocatalyst with a comparably high degree of amino acid amide racemase activity, and preferably one single microorganism expressing both activities to comparable degrees, offers a suitable method of preparing optically pure D-amino acids starting from D- or L-amino acid amides. The same holds, mutatis mutandis, for the combination of an L-amidase and an amino acid amide racemase, preferably expressed in one microorganism with comparably high activities, with regard to the preparation of optically pure L-amino acids from the above-mentioned raw materials.

An example of a microorganism expressing both amino acid amide racemase and amidase activity, is the above-mentioned strain of Pseudomonas putida NCIB 40042. This strain has been obtained according to the method described in the recently filed patent application NL-88.01864.

The inducible D-amidase activity is brought to expression by inoculating this strain in a culture medium in which a D-amino acid amide, preferably D-phenylglycine amide (whether or not as N-source) is present.

The expression of amino acid amide racemase activity together with L-amidase activity can be obtained by cultivating this Pseudomonas putida strain in the absence of a D-amino acid amide, as a result of which the D-amidase activity is not brought to expression.

The expression of amino acid amide racemase activity and D-amidase activity may possibly be obtained by, for example, preparing an L-amidase-negative mutant from the Pseudomonas putida strain using classical genetic techniques (such as UV-irradiation, or alkylating reagents) or by using recombinant DNA-techniques, and then cultivating it in the presence of a D-amino acid amide (whether or not as N-source). It is also possible to obtain, by enzyme purification, purified fractions with D-amidase activity on the one hand and amino acid amide racemase activity on the other. Combination of these fractions then yields a preferred biocatalyst for the preparation of optically active D-amino acids.

Besides the above-mentioned strain NCIB 40042, D-amidase can also be derived from Rhodococcus sp. strain NCIB 40041. This strain was deposited for patenting purposes on 2 August, 1988 under the terms of the Budapest Treaty with the National Collections of Industrial and Marine Bacteria Ltd.

Many microbial L-amidases are known and can be used in the practice of the invention. Besides the above-mentioned enzyme from strain NCIB 40042, an excellent example is the enzyme from Pseudomonas putida strain ATCC 12633.

The amidase activities can be derived from the indicated microorganisms in the same way as mentioned for the amide racemase.

The following examples are given for the purpose of illustrating the operation of the process of the invention without, of course, limiting in any manner to the examples:

Example 1

The Pseudomonas putida strain NCIB 40042 was cultivated overnight in a YCB (10 g/l) medium with D-phenylglycine amide as N-source (1 g/l). After harvesting of the cells by centrifugation and washing, each time 120 mg of cell sludge was added to 5 ml of a 1% (w/v) solution of D- or L-phenylglycine amide or D- or L-phenylglycine as substrate. The reaction was conducted for 20 hours in a small vessel at 37°C and pH 8.5, with shaking.

The several reactions yielded the following results:

| Substrate | Biocatalyst | Percentage conversion to D-phenylglycine and L-phenylglycine, respectively | |
|---|---|---|---|
| D-phenylglycine amide | present | 77% | 14% |
| L-phenylglycine amide | present | 7% | 89% |
| D-phenylglycine amide | absent | <1% | <1% |
| L-phenylglycine amide | absent | <1% | <1% |
| D-phenylglycine | present | -- | <1% |
| L-phenylglycine | present | <1% | -- |

The above results, which were calculated after chiral HPLC analysis of the reaction mixtures obtained, clearly show that, besides the prepared D-amidase activity, this biocatalyst has an amino acid amide racemase activity for phenylglycine amide. As indicated, racemization does not occur at the level of the amino acid produced. Only with the corresponding amide as substrate (both the D- and the L-phenylglycine amide) both D-phenylglycine and L-phenylglycine are formed in the simultaneous presence of the L-amidase and D-amidase activities.

Example 2

Pseudomonas putida strain NCIB 40042 was cultivated overnight at 28°C in a YCB (10 g/l) medium with D-phenylglycine amide as N-source (1 g/l). After harvesting of the cells by centrifugation and washing, the cells were disintegrated with the aid of a French-press, after which, by centrifugation, a cell-free extract was obtained.

Since many enzymatic racemization reactions are known to have pyridoxal-5′-phosphate (PLP) as a cofactor, it was investigated whether this is also the case with the racemization of amino acid amides.

If the amino acid amide racemase is indeed PLP-dependent, the reaction should be inhibited by an inhibitor specific to PLP-dependent reactions, e.g. hydroxylamine.

The reactions were carried out for 16 hours with D-phenylglycine amide 5 ml 1% (w/v) as substrate at 37°C and pH 8.5 and with 0.5 ml of the cell-free extract as biocatalyst. The following results were obtained:

| Hydroxylamine sulphate 0.2 mmol/ml | Reaction products D-phenylglycine | L-phenylglycine |
|---|---|---|
| absent | + | +++ |
| 10 µl | + | ++ |
| 50 µl | + | + |

In these racemization reactions, additional PLP results in increased amino acid amide racemase activity.

These results show that the racemization of amino acid amides is indeed a PLP-dependent reaction, which can be inhibited with the aid of hydroxylamine.

Example 3

Pseudomonas putida strain NCIB 40042 was cultivated overnight at 28°C in a 30 litre bioreactor (MBR), pH 7.1, with a medium volume of 20 litres, no D-amino acid amide (e.g. D-phenylglycine amide) having been added to this medium. The biocatalyst cultivated in this manner possesses exclusively L-amidase and amino acid amide racemase activity and no D-amidase activity. After harvesting of the cells by, successively, ultrafiltration, centrifugation, washing and freeze-drying, these cells were used as biocatalyst - in different ratios relative to the substrate - in the enzymatic reactions described below. As substrate, 50 ml of a 2% (w/v) D,L-phenylglycine amide solution, pH 8.6, was used. The reactions were carried out at 40°C. The percentage conversion was determined on the basis of the quantity of ammonia formed by the enzymatic hydrolysis of the amino acid amide into the corresponding amino acid. This determination of the ammonia concentration in the reaction medium was carried out with the aid of an ion-selective ammonia electrode (Orion).

| Biocatalyst mg | Enzyme/Substrate ratio | Reaction time hours | Percentage conversion of the substrate (amino acid amide) |
|---|---|---|---|
| 100 | 1:10 | 24 | 52% |
| | | 50 | 53% |
| | | 72 | 58% |
| 500 | 1:2 | 24 | 53% |
| | | 50 | 57% |
| | | 72 | 63% |
| 2000 | 2:1 | 24 | 65% |
| | | 50 | 74% |
| | | 72 | 80% |

Although these results clearly show that the amino acid amide racemase activity is the limiting enzymatic activity in the hydrolysis of the amino acid amide, it does show that by enzymatic racemization of the amino acid amide as substrate for the reaction a conversion percentage of more than 50% can be achieved. In addition, with chiral TLC analyses it was demonstrated that exclusively L-phenylglycine and no D-phenylglycine was formed, which makes this process excellently suitable for the preparation of optically pure L-phenylglycine.

Example 4

Since the conversion percentages obtained in the preparation process described in Example 3 require a long reaction time, it is possible that during this time D-amidase activity is induced, for D-phenylglycine amide is present in the reaction mixture besides L-phenylglycine amide. To prevent this, the same reaction was performed in the presence of a protein synthesis inhibitor, in this case tetracycline, in a concentration that ensures total inhibition (10 µg/ml). The reaction was carried out at 40°C and pH 8.15 and with a 2% (w/v) racemic D,L-phenylglycine amide mixture (40 ml) as substrate. In this way, the following results were obtained:

| Biocatalyst mg | Enzyme/Substrate ratio | Reaction time hours | Percentage conversion of the substrate |
|---|---|---|---|
| 403 | 1:2 | 17.5 | 52% |
| | | 47.5 | 56% |
| | | 72 | 58% |
| 1600 | 2:1 | 17.5 | 58% |
| | | 47.5 | 69% |
| | | 72 | 75% |

Since the conversion percentages thus obtained are at the same level as those in Example 3, where no protein-synthesis inhibitor was added to the reaction mixture, it must be concluded that the fact that the aforementioned conversion percentages are above 50 is due to the presence of an amino acid amide racemase.

Example 5

Since it should also be possible to obtain L-phenylglycine as product starting from optically pure D-phenylglycine amide as substrate, in the presence of a biocatalyst with amino acid amide racemase activity combined with L-amidase activity, this was done in an analogous experiment (cf. Examples 3 and 4). Cells of Pseudomonas putida strain NCIB 40042, cultivated in the same way as in Example 3, were used as biocatalyst (300 mg) in 30 ml of a 1% (w/v) aqueous solution of D-phenylglycine amide. The reaction was carried out at 40°C and pH 9.0. The percentage conversion was again determined via measurement of the quantity of ammonia produced, using an ion-selective ammonia electrode. The following results were obtained:

| Reaction time hours | Percentage conversion of the substrate |
|---|---|
| 17 | 9% |
| 43 | 26% |

This experiment, too, gives proof of the enzymatic racemization of an amino acid amide, in this case the optically pure D-phenylglycine amide, with simultaneous hydrolysis of the formed L-phenylglycine amide to optically pure L-phenylglycine.

Example 6

To determine the substrate specificity of the amino acid amide racemase two aromatic as well as two aliphatic D-amino acid amides were used as substrates (5 ml of 1% (w/v) aqueous solution) As biocatalyst, freeze-dried cells were used, which had been obtained in the same manner as described in Examples 3, 4 and 5. The reaction proceeded for 17 hours at 38°C and at a pH of 8.1. As aromatic amino acid amides, D-phenylglycine amide and D-homophenylalanine amide were used, and as aliphatic amino acid amides D-valine amide and D-leucine amide. In all cases, exclusively the corresponding L-amino acids were formed as reaction products, as analyzed with chiral TLC analysis. This is a proof of the broad substrate specificity of the amino acid amide racemase, which possesses a racemizing enzyme activity for aromatic amino acid amides as well as for aliphatic amino acid amides.

Example 7

Alaninonitrile was added to 5 ml of a phosphate buffer (0.1 M, pH 7.0) corresponding to a concentration of the nitrile of 100 mM. The reaction mixture was stirred at room temperature and 0.1 ml of a suspension of freshly fermented cells of Rhodococcus sp. NCIB 12569 was added to the reaction mixture. At 30 minutes intervals the concentrations of D- and L-alanine amide as well as D- and L-alanine were monitored using chiral

HPLC over a period of 4 hours. The following processes were observed to take place in the reaction mixture: hydrolysis of the alaninonitrile into the amide, racemization of the amide, and hydrolysis of the amide into alanine.

Example 8

L-Alanine amide (HBr salt, 11.4 mg) was added to 1.125 ml of phosphate buffer (0.1 M, pH 7.0) thereby yielding a solution of the amide of 60 mM. This solution was stirred at room temperature and freshly fermented cells of Rhodococcus sp. NCIB 12569 suspended in 0.225 ml of the same buffer were added to the reaction mixture. After 5, 10, 30, 60, and 120 minutes aliquots of the reaction mixture were withdrawn and assayed for their content of amino acids and amino acid amides by employing chiral HPLC. The concentrations of D- and L-alanine formed after 60 minutes of incubation were determined to 13.4 and 20.5 mM respectively. The amino acid racemase activity of the cells was measured by incubating the same quantity of cells with L-alanine from which only 3.5 mM of D-alanine were generated in the course of 60 minutes.

Example 9

A 60 mM solution of D-alanine amide hydrochloride was prepared by dissolving 8.4 mg of the amide in 1.125 ml of phosphate buffer (0.1 M, pH 7.0). After incubation with Rhodococcus sp. NCIB 12569 as indicated in the previous example the concentrations of D-and L-alanine detected after 60 minutes were 30.1 and 7.5 mM, respectively. Using an identical cell suspension 50 mM D-alanine was racemized to only 2.4 mM of L-alanine under the same conditions in the course of 60 minutes.

Example 10

A suspension of freshly fermented cells of Rhodococcus sp. NCIB 12569 in 0.25 ml of buffer was added to 1.125 ml of a 120 mM solution of racemic alanine amide in phosphate buffer (0.1M, pH 7.0). After 4 hours the conversion of the amide into alanine was complete. The enantiomeric excess of D-alanine (calculated as $\frac{D - L}{D + L}$ x 100%) was determined to 75%.

It is seen that D-alanine can be synthesized in good yield and high selectivity by exposure of the racemic amide to a D-amidase and an amino acid amide racemase.

Example 11

A suspension of freshly fermented cells of Rhodococcus sp. NCIB 12569 in 0.225 ml of buffer and 0.225 ml of a suspension of Pseudomonas putida cells exhibiting L-amidase activity as described in US-A-4,080,259 were added to a 140 mM solution of racemic alanine amide in phosphate buffer (0.1 M, pH 7.0). After 4 hours the reaction was complete. The enantiomeric excess of the L-alanine was found to be 80%.

Example 12

A 0.45 ml suspension of freshly fermented cells of the Rhodococcus sp. strain NCIB 12569 and strain NCIB 40041 (Novo strain 23, deposited at NCIB June 26, 1988) in phosphate buffer were added to a 140 mM solution of racemic valine amide in phosphate buffer (0.1 M, pH 7.0). The resulting mixture was stirred at room temperature and the progress of the reaction was monitored by chiral HPLC. After 6 hours the reaction was judged to be complete. The enantiomeric excess of D-valine was 90%. Thus, valine enriched in the L-isomer can be produced from the racemic amide by exposure to an L-amidase and an amino acid amide racemase.

## Claims

1. Process for complete or partial racemization of compounds of the general formula I

R - CH(NH2) - CONH2 (I)

wherin R represents indolyl, benzyloxy, lower alkyl, optionally substituted by hydroxy, mercapto, amino, halogen, phenyl, phenoxy, benzyl or lower alkylthio, or R represents phenyl optionally substituted by one or more of the following substituents: hydroxy, amino, halogen, carboxy or lower alkoxy characterized by exposing said compounds to an enzyme of microbial origin with amino acidamide racemase activity towards the amide in question.

2. Process according to claim 1, characterized by said enzyme being derived from a strain of Rhodococcus sp. or being derived from a strain of Pseudomonas.

3. Process according to claim 1, characterized by said enzyme being derived from a strain of Ps. putida.

4. Process according to claim 1 or 2, characterized by said enzyme being derived from strain NCIB 12569 or strain NCIB 40042.

5. Process according to any of the preceding claims, characterized in that the racemization is carried out in the presence of an enantioselective amino acid amidase with activity towards the amide in question.

6. Process according to claim 5, characterized by said enantioselective amidase being L-specific.

7. Process according to claim 6, characterized by said L-specific amidase being derived from a strain of Pseudomonas.

8. Process according to claim 5, characterized by said enantioselective amidase being D-specific.

9. Process according to claim 8, characterized by said D-specific amidase being derived from a strain of Rhodococcus sp. or a strain of Pseudomonas.

10. Process according to claim 5, characterized by the amide racemase and the enantioselective amidase activity being both expressed in one microorganism.

## Patentansprüche

1. Verfahren zur vollständigen oder teilweisen Racemisierung von Verbindungen der allgemeinen Formel I

$$R - CH(NH_2) - CONH_2 \qquad (I)$$

in der R Indolyl, Benzyloxy, Niederalkyl, gegebenenfalls substituiert durch Hydroxyl, Mercapto, Amino, Halogen, Phenyl, Phenoxy, Benzyl oder Niederalkylthio darstellt, oder R Phenyl darstellt, gegebenenfalls substituiert durch einen oder mehrere der folgenden Substituenten: Hydroxyl, Amino, Halogen, Carboxyl oder Niederalkoxy, **dadurch gekennzeichnet**, daß die Verbindungen einem Enzym mikrobieller Herkunft mit Aminosäureamid-Racemaseaktivität gegenüber dem in Rede stehenden Amid ausgesetzt werden.

2. Verfahren nach Anspruch 1, **dadurch gekennzeichnet**, daß das Enzym von einem Stamm von Rhodococcus sp. oder einem Stamm von Pseudomonas stammt.

3. Verfahren nach Anspruch 1, **dadurch gekennzeichnet**, daß das Enzym von einem Stamm von Ps. putida stammt.

4. Verfahren nach Anspruch 1 oder 2, **dadurch gekennzeichnet**, daß das Enzym vom Stamm NCIB 12569 oder vom Stamm NCIB 40042 stammt.

5. Verfahren nach einem der vorhergehenden Ansprüche, **dadurch gekennzeichnet**, daß die Racemisierung in Gegenwart einer enantioselektiven Aminosäureamidase mit Aktivität gegenüber dem in Rede stehenden Amid durchgeführt wird.

6. Verfahren nach Anspruch 5, **dadurch gekennzeichnet**, daß die enantioselektive Amidase L-spezifisch ist.

7. Verfahren nach Anspruch 6, **dadurch gekennzeichnet**, daß die L-spezifische Amidase von einem Stamm von Pseudomonas stammt.

8. Verfahren nach Anspruch 5, **dadurch gekennzeichnet**, daß die enantioselektive Amidase D-spezifisch ist.

9. Verfahren nach Anspruch 8, **dadurch gekennzeichnet**, daß die D-spezifische Amidase von einem

Stamm von Rhodococcus sp. oder von einem Stamm von Pseudomonas stammt.

10. Verfahren nach Anspruch 5, **dadurch gekennzeichnet**, daß sowohl die Amidracemase- als auch die enantioselektive Amidaseaktivität in einem Mikroorganismus exprimiert sind.

## Revendications

1. Procédé pour la racémisation totale ou partielle de composés de formule générale I

$$R - CH\ (NH_2) - CONH_2 \qquad (I)$$

où R représente un radical indolyle, benzyloxy, alkyle inférieur, éventuellement par un radical hydroxy, marcapto, amino, halogéno, phényle, phénoxy, benzyle ou alkyl(inférieur)thio, ou R représente un radical phényle éventuellement substitué par un ou plusieurs des substituants suivants: hydroxy, amino, halogéno, carboxy ou alcoxy inférieur, caractérisé en ce qu'on expose lesdits composés à une enzyme d'origine microbienne ayant une activité de racémase d'amide d'acide aminé vis-à-vis de l'amide en question.

2. Procédé selon la revendication 1, caractérisé en ce que ladite enzyme est dérivée d'une souche de Rhodococcus sp. ou est dérivée d'une souche de Pseudomonas.

3. Procédé selon la revendication 1, caractérisé en ce que ladite enzyme es dérivée d'une souche de Ps. putida.

4. Procédé selon l'une des revendications 1 ou 2, caractérisé en ce que ladite enzyme est dérivée d'une souche NCIB 12569 ou d'une souche NCIB 40042.

5. Procédé selon l'une quelconque des revendications précédentes, caractérisé en ce que la racémisation est effectuée en présence d'une amidase d'acide aminé énantiosélective ayant une activité vis-à-vis de l'amide en question.

6. Procédé selon la revendication 5, caractérisé en ce que ladite amidase énantiosélective est L-spécifique.

7. Procédé selon la revendication 6, caractérisé en ce que ladite amidase L-spécifique est dérivée d'une souche de Pseudomonas.

8. Procédé selon la revendication 5, caractérisé en ce que ladite amidase énantiosélective est D-spécifique.

9. Procédé selon la revendication 8, caractérisé en ce que ladite amidase D-spécifique est dérivée d'une souche de Rhodococcus sp. ou d'une souche de Pseudomonas.

10. Procédé selon la revendication 5, caractérisé en ce que l'activité de racémase d'amide et l'activité d'amidase énantiosélective sont toutes deux esprimées dans un même microorganisme.